# EUROPÄISCHE PATENTANMELDUNG

(11) **EP 1 974 768 A1**
(43) Veröffentlichungstag der Anmeldung: **01.10.2008**
(21) Anmeldenummer: 07023806.8
(22) Anmeldetag: 08.12.2007
(51) Int. Cl.: A61N 1/375

(54) **Dichtelement und Kontaktbuchse für medizinisches Implantat**

(30) Priorität: 24.03.2007 DE 102007014217
(71) Anmelder: BIOTRONIK CRM Patent AG, 6341 Baar (CH)
(72) Erfinder: Rebentisch, Ronald, 10967 Berlin (DE)
(74) Vertreter: Lindner-Vogt, Karin L.

(57) **Zusammenfassung**

Die Erfindung betrifft eine Kontaktbuchse zum Anschluss einer Elektrodenleitung an ein implantierbares medizinisches Gerät, mit einem Buchsengehäuse, welches eine Steckeraufnahme mit einem längsgestreckten Aufnahmeraum mit einer Längsachse zum Aufnehmen eines Steckers entlang der Längsachse aufweist, wobei die Steckeraufnahme von einem einstückigen, sich über die gesamte Länge der Steckeraufnahme erstreckenden Gussteil aus einer dauerelastischen Masse gebildet ist, in welches elektrische Kontaktelemente aus elektrisch leitendem Material eingesetzt sind.

## Beschreibung

Die Erfindung betrifft eine Kontaktbuchse zum Anschließen einer Elektrodenleitung an ein implantierbares medizinisches Gerät, insbesondere an einen implantierbaren Herzschrittmacher oder einen implantierbaren Kardioverter/Defibrillator.

Derartige Kontaktbuchsen besitzen ein Buchsengehäuse mit einer Steckeraufnahme mit einem längsgestreckten Aufnahmeraum zur Aufnahme eines passenden Steckers.

Bei Herzschrittmachern oder Defibrillatoren dient die Kontaktbuchse dazu, einen Stecker am proximalen Ende einer Elektrodenleitung einerseits dicht mit dem Gehäuse des Herzschrittmachers oder Defibrillators zu verbinden und gleichzeitig eine sichere und zuverlässige elektrische Verbindung herzustellen. Über den Stecker am proximalen Ende der Elektrodenleitung werden üblicherweise im Bereich des distalen Endes der Elektrodenleitung vorgesehene Elek-troden elektrisch mit elektrischen und/oder elektronischen Komponenten im Inneren des Gehäuses des Herzschrittmachers oder Defibrillators verbunden. Zu diesen elektrischen und/oder elektronischen Komponenten zählen beispielsweise Stimulationsimpulsgeneratoren oder Eingangsverstärker zur Verstärkung von im Herzen aufgenommenen elektrischen Potentialen.

Die Kontaktbuchse ist üblicherweise in einem Anschlussstück angeordnet, welches selbst aus isolierendem Kunststoff besteht und dicht mit einem übrigen Gehäuse eines Herzschrittmachers oder Defibrillators verbunden ist. Das übrige Gehäuse besteht üblicherweise aus Metall und schließt die elektrischen und/oder elektronischen Komponenten ein. Die elektrische Verbindung zwischen den Komponenten im Inneren des Gehäuses des Herzschrittmachers und dem Anschlussstück sowie der im Anschlussstück angeordneten Kontaktbuchse erfolgt über elektrische Durchführungen.

In der Kontaktbuchse selbst sind elektrisch leitende Kontaktelemente angeordnet, mit denen entsprechende Gegenkontakte des Steckers an der Elektrodenleitung kontaktiert werden, das heißt, elektrisch leitend verbunden werden können.

Um eine Austauschbarkeit von Elektroden bzw. Herzschrittmachern und Defibrillatoren verschiedener Hersteller zu ermöglichen, sind die Anschlussmaße des Steckers an der Elektrodenleitung sowie die entsprechenden Maße der Kontaktbuchse im Anschlussstück des Herzschrittmachers oder Defibrillators standardisiert. Der derzeit am weitesten verbreitete Standard wird üblicherweise mit IS-1 bezeichnet. Ein neuer, aktueller Standard ist unter der Bezeichnung IS-4 bekannt.

Der Erfindung liegt die Aufgabe zugrunde, eine Kontaktbuchse zu schaffen, die eine zuverlässige Herstellung eines Anschlussstücks, insbesondere für einen Herzschrittmacher oder Defibrillator erlaubt und die darüber hinaus eine zuverlässige elektrische Verbindung mit den Gegenkontakten am Stecker einer Elektrodenleitung gewährleistet oder die eine zuverlässige Abdichtung der Kontaktbuchse mit eingesetztem Elektrodenleitungsstecker gegenüber umgebenden Medien, wie beispielsweise Blut, gewährleistet.

Erfindungsgemäß wird diese Aufgabe durch eine Kontaktbuchse zum Anschluss an eine Elektrodenleitung an ein implantierbares medizinisches Gerät gelöst, die ein Buchsengehäuse auf einer Steckeraufnahme aufweist, in dessen längsgestreckten Aufnahmeraum ein Stecker einer Elektrodenleitung einzusetzen ist. Die Steckeraufnahme ist ein einstöckiges Gussteil, das sich über die gesamte Länge der Steckeraufnahme erstreckt und von einer dauerelastischen Masse gebildet ist. In die Steckeraufnahme sind elektrische Kontaktelemente aus elektrisch leitendem Material eingesetzt.

Das Gussteil ist vorzugsweise ein Spritzgussteil und die dauerelastische Masse besteht vorzugsweise aus Silikon oder enthält zumindest Silikon. Silikon besitzt eine geeignet einstellbare Elastizität und erleichtert wegen guter Gleiteigenschaften das Einführen eines Elektrodenleitungssteckers in den Aufnahmeraum.

Eine einstückige Steckeraufnahme vereinfacht die Herstellung einer Kontaktbuchse bzw. eines Anschlussgehäuses für einen implantierbaren Herzschrittmacher oder Kardioverter/Defibrillator und begünstigt allein schon deswegen die Zuverlässigkeit des Gerätes.

Darüber hinaus ist eine einstückige Steckeraufnahme vorteilhaft in Bezug auf die Dichtigkeit der Kontaktbuchse.

Der letztgenannte Aspekt kommt besonders dann zum Tragen, wenn der von der Steckeraufnahme eingeschlossene Aufnahmeraum nur an einem Längsende zum Einführen eines Steckers offen ist und am anderen Längsende geschlossen ist. Dementsprechend wird eine derartige Ausführungsvariante bevorzugt.

Die Dichtigkeit der Kontaktbuchse wird gemäß einer bevorzugten Ausführungsvariante weiterhin dadurch gefördert, dass das einstückige Gussteil bereits Dichtungsrippen enthält.

Die Dichtungsrippen sind vorzugsweise in unmittelbarer Nachbarschaft zu in die Steckeraufnahme eingesetzten Kontaktelementen angeordnet. Für diese Kontaktelemente sind in der Steckeraufnahme vorzugsweise Aufnahmeräume vorgesehen, so dass das einstückige Gussteil, in Längsrichtung gesehen, vorzugsweise abwechselnd Aufnahmeräume für jeweils ein Kontaktelement und dazwischen angeordnete Dichtungsrippen aufweist. Sämtliche Querschnitte der Steckeraufnahmen besitzen dabei vorzugsweise eine kreisrunde Grundform. Lediglich im Bereich der Kontaktaufnahmeräume sind vorzugsweise Durchgangsöffnungen vorgesehen, die sich, ausgehend vom jeweiligen Kontaktaufnahmeraum in Bezug auf die Steckeraufnahme nach außen hin öffnen. Dementsprechend besitzt die Steckeraufnahme und das einstückige Gussteil im Bereich dieser Durchgangsöffnungen keinen kreisförmigen Innenquerschnitt.

Der Außenquerschnitt des einstückigen Gussteils bzw. der Steckeraufnahme ist über die gesamte Länge der Steckeraufnahme vorzugsweise durchgehend kreisrund, so dass die Steckeraufnahme von außen betrachtet die Form eines Kreiszylinders besitzt.

Der Innendurchmesser des Gussteils und der Steckeraufnahme beträgt im Bereich der Dichtungsrippen weniger als 3,2 mm und ist im Übrigen größer als 3,2 mm.

Neben drei Kontaktaufnahmeräumen für Kontaktelemente zum Kontaktieren von Ringkontakten ist vorzugsweise im Bereich eines geschlossenen Endes der Steckeraufnahme ein Kontaktaufnahmeraum zur Aufnahme eines Terminal-Schraubkontaktes vorgesehen.

Wie bereits erwähnt, ist ein bevorzugtes Material für die Steckeraufnahme bzw. das die Steckeraufnahme bildende Gussteil Silikon, und zwar vorzugsweise mit einer Shore-Härte zwischen 30 und 70, bevorzugt 50. Das die Steckeraufnahme bildende Gussteil ist darüber hinaus vorzugsweise getempert, um eine gewünschte Härte sicherzustellen.

Darüber hinaus ist die aus einem elastischem Kunststoff bestehende Steckeraufnahme vorzugsweise von hartem Kunststoff umgeben. Beispielsweise kann die Steckeraufnahme mit Epoxidharz, das vorzugsweise gleich die äußere Kontur des Anschlussgehäuses definiert, umgeben sein.

Die Erfindung betrifft außerdem ein Verfahren zum Herstellen der zuvor beschriebenen Steckeraufnahme.

Dieses Verfahren umfasst die folgenden Verfahrensschritte:
- Bereitstellen einer äußeren Gussform und eines Gusskerns sowie Zusammenfügen derselben, so dass diese einen Freiraum definieren, der sowohl die Innen- als auch die Außenkonturen der Steckeraufnahme bestimmt,
- Auffüllen des Freiraums mit flüssigem Silikon,
- Aushärtenlassen des Silikons, welches somit die Steckeraufnahme bildet,
- Entfernen der äußeren Gussform nach Aushärten der Steckeraufnahme,
- Quellenlassen der Steckeraufnahme und
- Entfernen des Gusskerns bei gequollener Steckeraufnahme.

Durch das Quellenlassen der Steckeraufnahme weitet sich diese soweit, dass der Gusskern entfernt werden kann, ohne dass beispielsweise feine Dichtungsrippen des die Steckeraufnahme bildenden Gussteils aus Silikon zerstört werden. Gleichzeitig können bei gequollener Steckeraufnahme leicht die elektrischen Kontaktelemente in die jeweiligen Kontaktaufnahmeräume eingesetzt werden.

Ein geeignetes Mittel, die Steckeraufnahme quellen zu lassen, ist n-Heptan.

Die Steckeraufnahme wird vorzugsweise nach dem Aushärten getempert, und zwar entweder vor dem Quellenlassen oder noch besser nach dem Einsetzen der Kontaktelemente.

Nach dem Einsetzen der Kontaktelemente können diese über zu den Kontaktelementen führende Durchgangsöffnungen mit elektrischen Verbindungsleitungen kontaktiert werden. Anschließend kann die Steckeraufnahme mit einem harten Kunststoff, vorzugsweise mit Epoxidharz, umgossen werden.

Die Erfindung soll nun anhand eines Ausführungsbeispiels mit Bezug auf die Figuren näher erläutert werden.

Von den Figuren zeigen:
- Figur 1:: eine schematische Seitenansicht eines Herzschrittmachers;
- Figur 2:: eine Seitenansicht eines Elektrodenleitungssteckers;
- Figur 3:: einen Längsschnitt durch eine Kontaktbuchse des Herzschrittmacher aus Figur 1; und
- Figur 4:: einen Längsschnitt und eine Stirnansicht einer Steckeraufnahme für eine Kontaktbuchse, ähnlich der aus Figur 3;

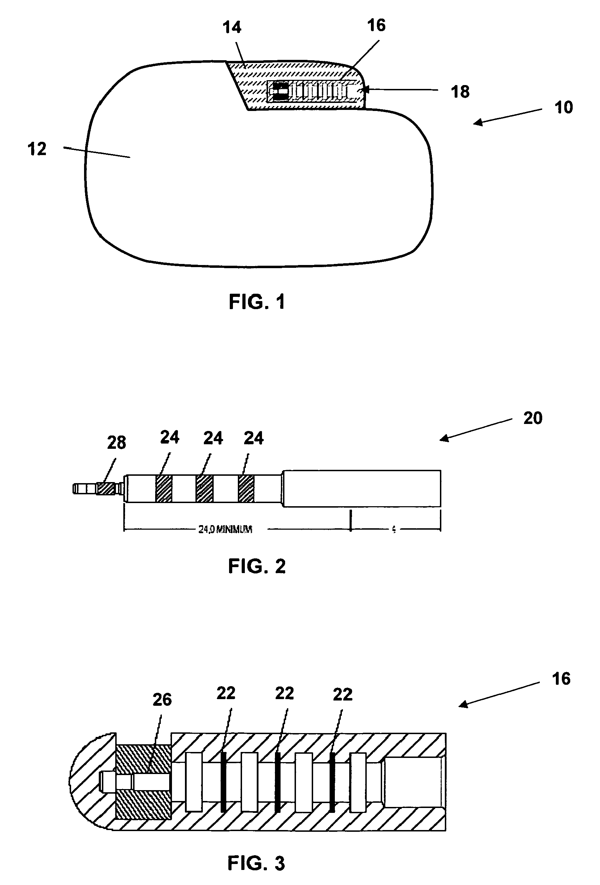

Figur 1 zeigt einen Herzschrittmacher 10 mit einem Metallgehäuse 12, das elektrische und/oder elektronische Komponenten des Herzschrittmachers beherbergt und einem dicht mit dem Metallgehäuse 12 verbundenen Anschlussgehäuse 14. Das Anschlussgehäuse 14 beherbergt eine Kontaktbuchse 16, deren wesentlicher Bestandteil ein Aufnahmeraum 18 zur Aufnahme eines Elektrodenleitungssteckers 20 ist, wie er in Figur 2 abgebildet ist. Figur 3 ist eine vergrößerte Ansicht der Kontaktbuchse 16 und ihres Aufnahmeraums 18 im Längsschnitt.

Die Kontaktbuchse weist drei gleichartige Kontaktelemente 22 auf, die dem Kontaktieren von ringförmigen Gegenkontaktflächen 24 des Elektrodenleitungssteckers 20 dienen.

Darüber hinaus weist die Kontaktbuchse 16 ein viertes Kontaktelement 26 auf, das zur Aufnahme eines Terminal-Schraubkontaktes 28 am äußeren freien Ende des Elektrodenleitungssteckers 20 dient.

Wesentlicher Bestandteil der Kontaktbuchse 16 ist neben den Kontaktelementen 22 und 26 eine Steckeraufnahme 30, die aus einem einstückigen Spritzgussteil aus Silikon besteht.

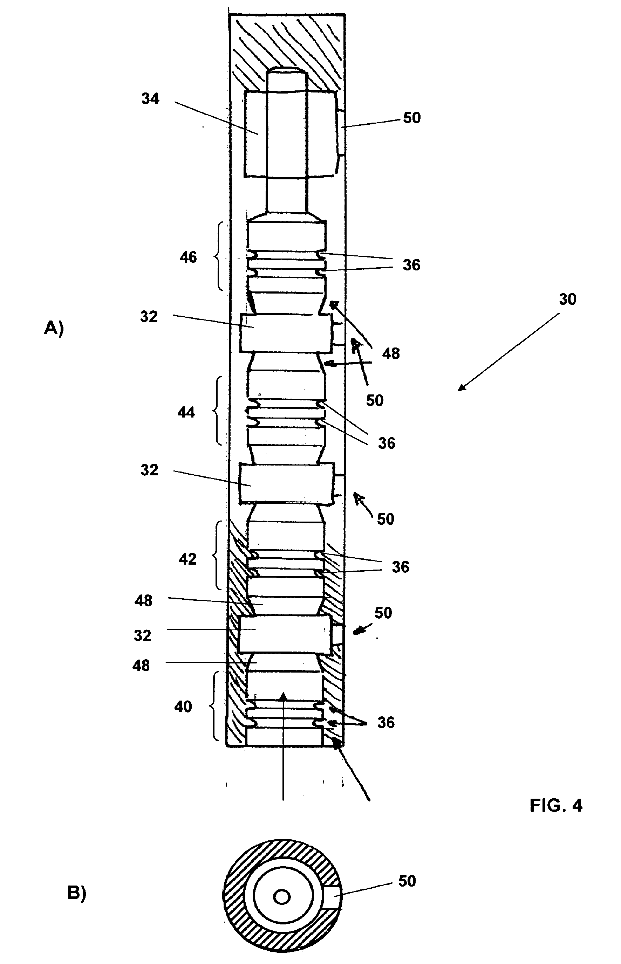

Figur 4 zeigt in Figur 4A einen Längsschnitt durch die Steckeraufnahme 30 und in Figur 4B eine Stirnansicht auf die offene Seite der Steckeraufnahme 30.

Wie Figur 4A zu entnehmen ist, weißt die Steckeraufnahme 30 insgesamt vier Kontaktaufnahmeräume auf, von denen drei Kontaktaufnahmeräume 32 der Aufnahme von Kontaktelementen für die Kontaktierung von Ringkontakten 24 eines Elektrodenleitungssteckers dienen, während der vierte Kontaktaufnahmeraum 34 zur Aufnahme eines Terminalschraubkontaktes dient.

Mit eingesetzten Kontaktelementen definiert die Steckeraufnahme 30 einen Aufnahmeraum 18, zur Aufnahme eines Steckers wie des Steckers 20 aus Figur 2. Der Aufnahmeraum 18 ist an einem Ende in der Nähe des Kontaktaufnahmeraums für den Terminal-Schraubkontakt geschlossen und am gegen-überliegenden Ende zum Einsetzen eines Steckers geöffnet.

Zwischen dieser Öffnung an einem Längsende des Aufnahmeraums 18 und des ersten Kontaktaufnahmeraums 32 sind zwei aufeinanderfolgende, ringförmige Dichtungen 36 vorgesehen, deren Lichtweite kleiner ist als 3,2 mm. Entsprechende Dichtungen 36 sind paarweise zwischen den Kontaktaufnahmeräumen 32 und 34 als integraler Bestandteil der einstückigen Kontaktaufnahme 30 vorgesehen.

Die Dichtungen 36 definieren einen ersten Dichtungsbereich 40, einen zweiten Dichtungsbereich 42, einen dritten Dichtungsbereich 44 und einen vierten Dichtungsbereich 46.

Die Kontaktaufnahmeräume 32 sind in Längsrichtung jeweils durch Fixations-elemente 48 für die einzusetzenden Kontaktelemente begrenzt. Von den Kontaktaufnahmeräumen 32 beziehungsweise 34 ausgehend, erstreckt sich jeweils eine Durchgangsöffnung 50 bis ins Äußere der Steckeraufnahme 30 und erlaubt es, das jeweilige Kontaktelement nach Einsetzen in die Steckeraufnahme 30 von Außen her mit einer elektrischen Leitung zu kontaktieren.

Die Steckeraufnahme 30 ist als einstückiges Gussteil aus Silikon im Spritzgussverfahren hergestellt. Der von der Steckeraufnahme 30 eingeschlossene Innenraum wird dadurch durch einen Gusskern vorgegeben, der nach dem Spritzgießen und Aushärten des Silikons dadurch entfernt wird, dass die Steckeraufnahme mit zunächst noch eingesetztem Gusskern (aber im Übrigen entfernter Gussform) in n-Heptan gebadet wird, so dass das Silikon quillt und sich der Innenraum der Steckeraufnahme 30 derart weitet, dass der Gusskern entfernt werden kann, ohne dass beispielsweise die feinen, rippenartigen Dichtungen 36 dabei beschädigt werden. In diesem gequollenen Zustand der Steckeraufnahme 30 können auch die Kontaktelemente in die Kontaktaufnahmeräume 32 beziehungsweise 34 eingesetzt werden.

Nach dem Einsetzen der Kontaktelemente kann die Steckeraufnahme 30 wieder schrumpfen, in dem das n-Heptan verdunstet. Anschließend wird die gesamte Steckeraufnahme 30 mit eingesetzten Kontaktelementen getempert, um die Vernetzung des bereits ausgehärteten Silikons zu erhöhen und somit eine gewünschte Materialhärte und Elastizität einzustellen.

Hiernach können die Kontaktelemente von Außen mit elektrischen Leitungen kontaktiert werden und anschließend die Steckeraufnahme mit eingesetzten und kontaktierten Kontaktelementen mit Epoxidharz umgossen werden, so dass im Ergebnis das in Figur 1 abgebildete Anschlussgehäuse 14 entsteht.

## Patentansprüche

1. Kontaktbuchse (16) zum Anschluss einer Elektrodenleitung an ein implantierbares medizinisches Gerät, mit einem Buchsengehäuse, welches eine Steckeraufnahme (30) mit einem längsgestreckten Aufnahmeraum (18) mit einer Längsachse zum Aufnehmen eines Steckers entlang der Längsachse aufweist, wobei die Steckeraufnahme (30) von einem einstückigen, sich über die gesamte Länge der Steckeraufnahme erstreckenden Gussteil aus einer dauerelastischen Masse gebildet ist, in welches elektrische Kontaktelemente aus elektrisch leitendem Material eingesetzt sind.

2. Kontaktbuchse nach Anspruch 1, **dadurch gekennzeichnet, dass** das einstückige Gussteil Dichtungsrippen (36) aufweist.

3. Kontaktbuchse nach Anspruch 1 oder 2, **dadurch gekennzeichnet, dass** die dauerelastische Masse Silikon enthält oder aus Silikon besteht.

4. Kontaktbuchse nach Anspruch 3, **dadurch gekennzeichnet, dass** die Steckeraufnahme aus Silikon mit einer Shore-Härte zwischen 30 und 70, bevorzugt 50, besteht und von einem vergleichsweise harten transparenten Kunststoff des Buchsengehäuses (14) umgeben ist.

5. Kontaktbuchse nach einem der Ansprüche 1 bis 4, **dadurch gekennzeichnet, dass** der harte transparente Kunststoff Epoxidharz enthält oder ist.

6. Kontaktbuchse nach einem der Ansprüche 1 bis 5, **dadurch gekennzeichnet, dass** die Steckeraufnahme (30) ein Spritzgussteil ist.

7. Kontaktbuchse nach Anspruch 6, **dadurch gekennzeichnet, dass** das Spritzgussteil getempert ist.

8. Kontaktbuchse nach einem der Ansprüche 1 bis 7, **dadurch gekennzeichnet, dass** die Steckeraufnahme (30) auf Ihrer Außenseite eine Kreiszylinderform hat.

9. Kontaktbuchse nach einem der Ansprüche 1 bis 8, **dadurch gekennzeichnet, dass** die Steckeraufnahme (30) über den größten Teil Ihrer Länge einen lichten Durchmesser von mehr als 3,2 mm besitzt und in Dichtungs-Längsabschnitten einen Durchmesser von weniger als 3,2 mm.

10. Kontaktbuchse nach einem der Ansprüche 1 bis 8, **dadurch gekennzeichnet, dass** die Steckeraufnahme (30) Kontaktaufnahmeräume (32, 34) aufweist, die sich jeweils über einen Bruchteil der Gesamtlänge des Aufnahmeraums erstrecken und die einen vergrößerten Innendurchmesser aufweisen, so dass ein jeweiliger Kontaktaufnahmeraum ein elektrisches Kontaktelement aus elektrisch leitendem Material aufnehmen kann.

11. Kontaktbuchse nach Anspruch 2 und 10, **dadurch gekennzeichnet, dass** die Dichtungsrippen (36) in Längsrichtung der Steckeraufnahme (30) gesehen, zwischen den Kontaktaufnahmeräumen (32, 34) angeordnet sind.

12. Kontaktbuchse nach Anspruch 10 oder 11, **dadurch gekennzeichnet, dass** die Steckeraufnahme (30) im Bereich der Kontaktaufnahmeräume (32, 34) jeweils wenigstens eine Durchgangsöffnung (50) aufweist, die vom jeweiligen Kontaktaufnahmeraum (32, 34) nach außen führt.

13. Kontaktbuchse nach einem der Anspruch 10 bis 12, **dadurch gekennzeichnet, dass** die Steckeraufnahme (30) insgesamt vier Kontaktaufnahmeräume (32, 34) aufweist, von denen drei (32) zur Aufnahme elektrischer Ringkontakte und einer (34) zur Aufnahme eines Terminal-Schraubkontaktes ausgebildet ist.

14. Kontaktbuchse nach einem der Ansprüche 1 bis 13, **dadurch gekennzeichnet, dass** die Kontaktbuchse (18) Teil eines Anschlussstücks (14) eines Herzschrittmachers (10) und/oder eines Kardioverters/Defibrillators ist.

15. Verfahren zum Herstellen einer Steckeraufnahme gemäß eines der Ansprüche 1 bis 14, **dadurch gekennzeichnet, dass** eine äußere Gussform und ein Gusskern bereitgestellt und so miteinander verbunden werden, dass sie einen Freiraum definieren, der die Innen- und Außenkonturen der Steckeraufnahme bestimmt,
dass anschließend der Freiraum mit flüssigem Silikon gefüllt wird,
dass das Silikon aushärten gelassen wird und somit die Steckeraufnahme bildet,
dass nach dem Aushärten die äußere Gussform entfernt wird,
dass die Steckeraufnahme anschließend quellen gelassen wird, und
dass schließlich der Gusskern entfernt wird.

16. Verfahren nach Anspruch 15, **dadurch gekennzeichnet, dass** zum Quellenlassen der Steckeraufnahme n-Heptan verwendet wird.

17. Verfahren nach Anspruch 15 oder 16, **dadurch gekennzeichnet, dass** in Kontaktaufnahmeräume der Steckeraufnahme in deren gequollenem Zustand jeweils ein Kontaktelement eingesetzt wird.

18. Verfahren nach einem der Ansprüche 15 bis 17, **dadurch gekennzeichnet, dass** die Steckeraufnahme nach dem entfernen des Gussteils bzw. nach dem Einsetzen der Kontaktelemente solange bei entsprechender Temperatur getempert wird, bis sich ein vorgegebener Endzustand einstellt.

19. Verfahren nach Anspruch 17 oder Anspruch 17 und 18, **dadurch gekennzeichnet dass** die Kontaktelemente nach dem Einsetzen in die Kontaktaufnahmeräume mit elektrischen Leitungen verbunden werden.

20. Verfahren nach Anspruch 19, **dadurch gekennzeichnet, dass** die Steckeraufnahme nach Einsetzen der Kontaktelemente und Anschließen der elektrischen Leitungen mit einem vergleichsweise harten Kunststoff umgossen wird.
